# EUROPEAN PATENT APPLICATION

(11) **EP 2 025 693 A1**
(43) Date of publication of application: **18.02.2009**
(21) Application number: 08013043.8
(22) Date of filing: 19.07.2008
(51) Int. Cl.: C08G 18/10, C08G 18/28, C08G 18/32, C08G 18/48, C08G 18/50, C08G 18/76, C08G 18/78, C08G 18/66

(54) **Allophanate modified diphenylmethane diisocyanates, prepolymers thereof, and their use in the preparation of polyureas and polyureaurethanes**

(30) Priority: 27.07.2007 US 881611
(71) Applicant: Bayer MaterialScience LLC, Pittsburgh, PA 15205 (US)
(72) Inventor: Johnston, Jay A., Pittsburgh PA 15205 (US); Haider, Karl W., Wexford PA 15090 (US); Gustavich, Wendy S., Box 383 Maynard OH 43937 (US)
(74) Representative: Klimiuk, Meike

(57) **Abstract**

This invention relates to novel allophanate-modifed diphenylmethane diisocyanates, prepolymers of these allophanate-modified diphenylmethane diisocyanates, and two-component polyureas and one-component polyureaurethanes prepared from these prepolymers. In addition, the invention also relates to processes for the preparation of these various compositions, and to the preparation of sealants from the two-component polyureas and caulking agents from the one-component polyureaurethanes.

## Description

### BACKGROUND OF THE INVENTION

This invention relates to novel allophanate modified diphenylmethane diisocyanates, and prepolymers of these allophanate modified diphenylmethane diisocyanates. It is also related to polyureas and polyureaurethanes comprising the prepolymers of these allophanate modified diphenylmethane diisocyanates.

Modified isocyanates, including modified diphenylmethane diisocyanates are known and described in the art. Various modifications include, for example, allophanate modified, biuret modified, carbodiimide modified, isocyanurate modified, uretdione modified, urethane modified, oxadiainetrione modified and various combinations thereof.

Allophanate modified diphenylmethane diisocyanates, prepolymers thereof, etc. are known and described in, for example, U.S. Patents 5,319,053 and 5,319,054. The liquid stable products of U.S. 5,319,053 are characterized by an NCO group content of 12 to 32.5%, and comprise the reaction product of an aliphatic alcohol and a specified mixture of isomers of diphenylmethane diisocyanate. This reference also discloses stable liquid MDI prepolymers which comprise the reaction product of the allophanate-modified MDI as described above, with an organic material containing two or more active hydrogen groups. U.S. Patent 5,319,054 describes liquid allophanate modified MDI compositions which are storage stable at 25°C. The diphenylmethane diisocyanate has a specific isomer distribution requiring 2 to 60% by weight of 2,4'-diphenylmethane diisocyanate.

Allophanate modified diphenylmethane diisocyanate prepolymers are described in U.S. Patent 5,440,003. These products are stable liquids diphenylmethane diisocyanate and an aromatic alcohol such as phenol, with the resultant product being converted to the allophanate having an NCO group content of 12 to 32% by weight.

Liquid polyisocyanate compositions are disclosed in EP 0031650. These polyisocyanate compositions are prepared by reacting diphenylmethane diisocyanate which contains at least 15% by weight of the 2,4'-isomer, with a monohydric alcohol or a monoalkoxy glycol. The highest molecular weight monoalkoxy glycol used to prepare an allophanate-modified isocyanate in the working examples is a monomethoxy polypropylene glycol having a molecular weight of 406.

Other patents which describe various allophanate-modified dipehnylmethane diisocyanates and prepolymers thereof, and optionally other modifications include, for example, U.S. Patents 4,738,991, 5,663,272, 5,783,652, 6,242,556, 6,482,913, 6,639,040, 6,838,542, 6,887,399 and 6,991,746. GB 994,980 also provides a general description of allophanate-modified isocyanates.

Advantages of the present invention include lower viscosities of the prepolymers based on these novel allophanate modified isocyanates. These lower viscosities allow for better mixing between isocyanate and polyol components, and the physical properties of the elastomers prepared from these are improved.

### SUMMARY OF THE INVENTION

This invention relates to allophanate-modified diphenylmethane diisocyanates, prepolymers of the allophanate modified diphenylmethane diisocyanates, to polyureas and polyureaurethanes which comprise these prepolymers, and to processes for the preparation of these.

The novel allophanate-modified diphenylmethane diisocyanates have an NCO group content of 0.25 to 30%, and comprise the reaction product of:
(a) diphenylmethane diisocyanate,
   with
(b) a polyether monol having an equivalent weight of greater than 1000 up to about 10,000;
   in the presence of :
(c) a suitable catalyst.

These novel allophanate-modifed diphenylmethane diisocyanates are prepared by reacting (a) diphenylmethane diisocyanate, with (b) a polyether monol having an equivalent weight of greater than 1000 up to about 10,000; in the presence of (c) a suitable catalyst.

The prepolymers of these allophanate-modified diphenylmethane diisocyanates have an NCO group content of 0.25% to 26% and comprise the reaction product of:
(1) an allophanate-modified diphenylmethane diisocyanate having an NCO group content of 0.25 to 30% and which comprises the reaction product of:
   (a) diphenylmethane diisocyanate,
      with
   (b) a polyether monol having an equivalent weight of greater than 1000 up to about 10,000;
      in the presence of
   (c) a suitable catalyst;
   and
(2) at least one polyether polyol having a functionality of from 1.5 to 6 and a molecular weight of from 500 to 10,000.

These prepolymers are prepared by reacting (1) the allophanate-modified diphenylmethane diisocyanates as described herein with (2) at least one polyether polyol having a functionality of from 1.5 to 6 and a molecular weight of from 500 to 10,000.

The two-component, amine-cured polyureas of the present invention comprise the reaction product of:
(A) a prepolymer of an allophanate-modified diphenylmethane diisocyanate in which the prepolymer has an NCO group content of 0.25 to 26% by weight and comprises the reaction product of:
   (1) an allophanate-modified diphenylmethane diisocyanate having an NCO group content of 0.25 to 30% by weight and which comprises the reaction product of:
      (a) diphenylmethane diisocyanate,
         with
      (b) a polyether monol having an equivalent weight of greater than 1000 up to about 10,000;
         in the presence of
      (c) a suitable catalyst;
      and
   (2) at least one polyether polyol having a functionality of from 1.5 to 6 and a molecular weight of from 500 to 10,000;
      and
(B) an isocyanate-reactive component comprising at least one compound selected from the group consisting of:
   (1) one or more amine group containing compounds having a functionality of at least 1.8 and a molecular weight of greater than 750 to about 7000;
      and
   (2) at least one diamine or polyamine having a molecular weight of less than or equal to 750.

These two-component, amine-cured polyureas are prepared by reacting (A) the prepolymer of the allophanate-modified diphenylmethane diisocyanate as described above, with (B) an isocyanate-reactive component selected from the group consisting of (1) one or more amine group containing compounds having a functionality of at least 1.8 and a molecular weight of greater than 750 to about 7000; and (2) at least one diamine or polyamine having a molecular weight of less than or equal to 750.

The one-component, moisture cured polyureaurethanes of the invention comprise the reaction product of:
(A) a prepolymer of an allophanate-modified diisocyanate having an NCO group content of 0.25 to 26% and comprising the reaction product of:
   (1) an allophanate-modified diphenylmethane diisocyanate having an NCO group content of 0.25 to 30% and which comprises the reaction product of:
      (a) diphenylmethane diisocyanate,
         with
      (b) a polyether monol having an equivalent weight of greater than 1000 up to about 10,000;
         in the presence of
      (c) a suitable catalyst;
         and
   (2) at least one polyether polyol having a functionality of from 1.5 to 4 and a molecular weight of from 500 to 10,000;
      and
(B) water;
   optionally, in the presence of
(C) one or more catalysts.

The process for preparing these one-component, moisture cured polyureaurethanes comprises reacting (A) a prepolymer of the allophanate-modified diphenylmethane diisocyanate as described herein, with (B) water, optionally, in the presence of (C) one or more catalyst.

The present invention also relates to coatings and elastomers prepared from the prepolymers of allophanate-modified isocyanates described herein, to sealants which comprise the polyureas described herein and to caulking agents which comprise the polyureaurethanes described herein.

### DETAILED DESCRIPTION OF THE INVENTION

This invention relates to allophanate-modified diphenylmethane diisocyanates having an NCO group content of about 0.25 to about 30%. In general, these allophanate-modified isocyanates have an NCO group content of at least about 0.25%, preferably at least about 1% and more preferably at least about 6.5%. These allophanate-modified isocyanates also have an NCO group content of less than or equal to 30%, preferably less than or equal to 26% and more preferably less than or equal to 19%. In addition, the allophanate-modified isocyanates may have an NCO group content ranging between any combination of these upper and lower values, inclusive, e.g., from 0.25 to 30%, preferably from 1 to 26% and more preferably from 6.5 to 19%.

The allophanate-modified diphenylmethane diisocyanates comprise the reaction product of: (a) diphenylmethane diisocyanate, with (b) a polyether monol having an equivalent weight of greater than 1000 up to 10,000; in the presence of (c) a suitable catalyst.

Suitable diphenylmethane diisocyanates to be used as (a) for the allophanate-modified diphenylmethane diisocyanates include any isomeric mixtures of diphenylmethane diisocyanate. In general, it is preferred to use a mixture comprising (i) from 1 to 81% by weight of the 2,4'-isomer, (ii) from 19 to 99% by weight of the 4,4'-isomer and (iii) from 0 to 6% by weight of the 2,2'-isomer, with the %'s by weight of (i), (ii) and (iii) totaling 100% by weight of the diphenylmethane diisocyanates. More preferably the mixture comprises (i) from 20 to 73% by weight of the 2,4'-isomer, (ii) from 27 to 80% by weight of the 4,4'-isomer and (iii) from 0 to 3% by weight of the 2,2'-isomer. Most preferably, the diphenylmethane (i) from 30 to 63% by weight of the 2,4'-isomer, (ii) from 37 to 70% by weight of the 4,4'-isomer and (iii) from 0 to 3% by weight of the 2,2'-isomer diisocyanate. The sum of the %'s by weight of (i), (ii) and (iii) always totals 100% by weight of diphenylmethane diisocyanate.

Suitable polyether monols for the allophanate-modified diphenylmethane diisocyanate include those having equivalent weights greater than 1000 and up to about 10,000. Typically, these polyether monols have equivalent weights of greater than 1000, preferably at least 1100 and more preferably at least 1200. These polyether monols also typically have equivalents weights of less than or equal to 10,000, preferably less than or equal to 7000 and more preferably less than or equal to 4500. Suitable polyether monols may have equivalents weights ranging between any combination of these equivalents weights (inclusive unless otherwise noted), e.g., greater than 1000 to less than or equal to 10,000, preferably at least 1100 to less than or equal to 7000, and more preferably at least 1200 to less than or equal to 4500.

As used herein, the term polyether monol refers to compounds of the above specified equivalent weight range which have a theoretical functionality ranging from about 1.0 to about 1.2.

Suitable polyether monols suitable for preparing the allophanate-modified diphenylmethane diisocyanates include, for example, those monols having equivalent weights and theoretical functionalities as set forth above, and are prepared according to well-known methods by condensing an alkylene oxide or a mixture of alkylene oxides using random or step-wise addition, with a hydric initiator or a mixture of such initiators. Illustrative alkylene oxides include, for example, ethylene oxide, propylene oxide, butylene oxide, amylene oxide, hexylene oxide, aralkylene oxides such as styrene oxide, and the halogenated alkylene oxides such as trichlorobutylene oxide and so forth. The more preferred alkylene oxide is propylene oxide or a mixture thereof with ethylene oxide, using either random or step-wise oxyalkyation.

Suitable hydric initiators (or starters) used for preparing the polyether monols herein include, for example, aromatic initiators such as phenol, benzyl alcohol, alkyl substituted phenols such as nonylphenol, etc., cycloaliphatic initiators such as cyclohexanol, alkyl substituted cyclohexnols, cyclopentanol, cyclohexylmethanol, etc., and aliphatic alcohols as initiators. Examples of suitable aliphatic alcohols include lower aliphatic alcohols having from 1 to 5 carbon atoms, and higher aliphatic alcohols having from 6 or more carbon atoms. The higher aliphatic alcohols include both the plasticizer range alcohols which contain from 6 to 11 carbon atoms, and the detergent range alcohols which contain 12 or more carbon atoms. Some examples of suitable aliphatic alcohols to be used in preparing the polyether monols herein include methanol, ethanol, propanol, 1- and 2-butanol, 1-pentanol, 1-hexanol, 2-methyl-1-pentanol, 4-methyl-2-pentanol, 2-ethyl-1-butanol, 1-heptanol, 1- octanol, 2-octanol, 2-ethylhexanol, 3,5-dimethyl-1-hexanol, 2,2,4-trimethyl-1-pentanol, 1-nonanol, 2,6-dimethyl-4-heptanol, 1-decanol, 1-undecanol, 1-dodecanol, 1-tridecanol, 1-tetradecanol, 1-pentadecanol, 1-hexadecanol, 1-heptadecanol, 1-octadecanol, 1-nonadecanol, 1-eicosanol, 1-hexacosanol, 1-heptatricontanol, etc., as well as mixtures thereof.

Also suitable to be used as initiators are compounds which contain one hydroxyl group and one or more double bonds such as, for example, allyl alcohol, 2-allylphenol, 2-allyl-6-methylphenol, cinamyl alcohol, undecelenyl alcohols, allylamines, acrylic acids, undecylenic acid, 9-hexadecen-1-ol, 9-octadecen-1-ol, 10-eicosen-1-ol, etc. In addition, mixtures of various alcohols may be used as the initiator for the polyether monols herein.

In addition to the monofunctional compounds, the initiator may also include a small amount of a di- or higher functional compound. Examples of these include ethylene glycol, propylene glycol, etc.

Particularly preferred initiators for preparing the polyether monols of the invention are nonylphenol, and mixtures of C₁₂ to C₁₅ alcohols which are commercially available as NEODOL®25 from Shell Chemical Company.

The alkoxylation of these suitable starter compounds may be performed by a suitable method such as, for example, by base catalysis utilizing strong bases such as sodium hydroxide, potassium hydroxide, sodium or potassium methoxide, etc. Other suitable catalysts include diethylzinc, combinations of metal naphthenates and tertiary amines, and the like. Preferred catalysts are double metal cyanide (DMC) complex catalysts such as, for example, hexacyanocobaltate.glyme catalysts which are disclosed in U.S. Patents 4,843,054 and 5,158,922, the disclosures of which are hereby incorporated by reference, and more preferably the substantially amorphous zinc hexacyanocobaltate t-butyl alcohol complex catalysts as disclosed in U.S. Patent 5,470,813, the disclosure of which is hereby incorporated by reference. It is particularly preferred to use the substantially amorphous zinc hexacyanocobaltate t-butyl alcohol complex catalysts to produce monodisperse polyoxyalkylene monols with exceptionally narrow molecular weight distributions.

Particularly preferred polyether monols are the propoxylation products of nonylphenol, or propoxylation products of mixtures of C₁₂ to C₁₅ alcohols (including those which are commercially available as NEODOL®25 from Shell Chemical Co.), which are prepared in the presence of a DMC catalyst.

Suitable catalysts to be used as component (c) herein include any of the known catalysts suitable for forming allophanates. Such catalysts include, but are not limited to, for example, zinc acetylacetonate, zinc 2-ethylhexanoate, cobalt 2-ethylhexanoate, cobalt naphthenate, lead linoresinate, etc.

The allophanate-modified diphenylmethane diisocyanates of the present invention are prepared in accordance with known processes as described in, for example, U.S. Patents 5,319,053, 5,319,054 and 5,440,003, the disclosures of which are hereby incorporated by reference. If necessary, catalyst stoppers as described therein are used in the present compositions and process for preparing these compositions. Benzoyl chloride is a preferred catalyst stopper.

Suitable prepolymers of the above described allophanate-modified diphenylmethane diisocyanates typically have NCO group contents of from 0.25 to 26%. In general, these prepolymers have NCO group contents of at least about 0.25%, preferably at least about 0.5% and more preferably at least about 1%. These prepolymers also typically have NCO group contents of less than or equal to 26, preferably less than or equal to 23%, and more preferably less than or equal to 16%. In addition, the prepolymers may have an NCO group content ranging between any combination of these upper and lower values, inclusive, e.g., from 0.25 to 26%, preferably from 0.5 to 23% and more preferably from 1 to 16%.

The prepolymers comprise the reaction product of (1) the allophanate-modified diphenylmethane diisocyanates as described above, with (2) at least one polyether polyol having a functionality of from 1.5 to 6 and a molecular weight of from 500 to 10,000.

For the prepolymers, the suitable (1) allophanate-modified diphenylmethane diisocyanates have NCO group contents as described above. The suitable (a) diphenylmethane diisocyanates for (1) the allophanate-modified diphenylmethane diisocyanates also have the isomer distribution as described above.

Suitable polyether monols to be used as (b) in preparing (1) the allophanate-modified diphenylmethane diisocyanates for (A) the prepolymers have equivalent weights within the ranges as described above.

Suitable polyether polyols to be used as component (A)(2) in the prepolymers of the allophanate-modified diphenylmethane diisocyanates typically have a functionality of from 1.5 to 6. In general, these polyether polyols will have a functionality of at least 1.5, and preferably of at least 1.8. The functionality of suitable polyether polyols is typically 6 or less, preferably 4 or less, more preferably 3.5 or less and most preferably 2.2 or less. Suitable polyether polyols may have functionalities ranging between any combination of these functionalities (inclusive), e.g. from 1.5 to 6, preferably from 1.5 to 4, more preferably from 1.5 to 3.5 and most preferably from 1.8 to 2.2.

These polyether polyols typically have molecular weights ranging from 500 to 10,000. In general, these polyether polyols will have molecular weights of 500, preferably at least 1000, and more preferably at least 1250, and most preferably at least 1500. These polyether polyols also typically have molecular weights of less than or equal to 10,000, preferably less than or equal to 7000, more preferably less than or equal to 5000, and most preferably less than or equal to 4500. Suitable polyether polyols may have molecular weights ranging between any combination of these molecular weights (inclusive), e.g., from 500 to less than or equal to 10,000, preferably at least 1000 to less than or equal to 7000, more preferably at least 1250 to less than or equal to 5000, and most preferably at least 1500 to less than or equal to 4500.

Suitable polyether polyols to be used herein include those known and typically used in polyurethane chemistry. Suitable polyether polyols include, for example, those prepared from a suitable initiator or starter compound having a suitable functionality for the desired polyether polyol, and alkoxylating the initiator with one or more alkylene oxides in the presence of a suitable catalyst to yield the desired polyether polyol. The alkylene oxides may be used individually, in mixtures with one another, and/or sequentially. Suitable intiators or starter compounds include, for example, ethylene glycol, propylene glycol, butylene glycol, trimethylolpropane, pentaerythritol, sorbitol, diethylene glycol, dipropylene glycol, dibutylene glycol, etc. Suitable alkylene oxides include ethylene oxide, propylene oxide, butylene oxide, styrene oxide, epichlorohydrin and tetrahydrofuran. Suitable catalysts include, for example, KOH, BF₃, DMC, etc.

A polyether polyol prepared from propylene glycol with propylene oxide, and having a molecular weight of 2000 and a functionality of 2 is most particularly preferred.

In accordance with the present invention, it is also possible to include one or more catalysts in the reaction between the polyether polyol and the allophanate-modified diphenylmethane diisocyanate to promote reaction between the NCO and OH groups in the formation of the prepolymers. This is optional in the present invention, although it may be desirable. When a catalyst is used or desired, any of the known and conventional catalysts from polyurethane chemistry would be suitable. Thus, both amine and alkanolamine compounds and organometallic compounds are suitable. Some examples include catalysts such as triethylamine, dimethylbenzeneamine, dicyclohexylamine, N,N,N',N'-tetramethyldiamino-diethylether, N,N'-dimorpholinodiethyl ether, N,N,N',N'-tetramethylethylenediamine, dimethylaminoethanol, N,N',N-tris(dimethylaminopropyl)-s-hexahydrotriazine, metal chlorides and metal salts such as iron(II) chloride, zinc chloride, lead octoate, tin dioctoate, tin diethyl-hexoate, dibutyltin dilaurate, dibutyldilauryltin mercaptide, as well as catalysts based on titanium, bismuth, zirconium, etc. In addition, ammonium hydroxides and alkali metal hydroxides may also be used as catalysts.

Prepolymers of the allophanate-modified diphenylmethane diisocyanates of the present invention are also prepared in accordance with known processes as described in, for example, U.S. Patents 5,319,053, 5,319,054 and 5,440,003, the disclosures of which are hereby incorporated by reference. If necessary, catalyst stoppers as described therein are used in the present compositions and process for preparing these compositions. Benzoyl chloride is a preferred catalyst stopper.

Suitable two-component, amine-cured polyureas of the present invention comprise the reaction product of (A) the above described prepolymer of an allophanate-modified diphenylmethane diisocyanate, and (B) an isocyanate-reactive component comprising at least one compound selected from the group consisting of (1) one or more amine group containing compound having a functionality of at least 1.8 and a molecular weight of greater than 750 to about 7000, and (2) at least one diamine or polyamine having a molecular weight of less than or equal to 750.

The polyureas of the present invention are prepared from prepolymers of allophanate-modified diphenylmethane diisocyanates. These prepolymers have an NCO group contents as described herein above. Typically, these range from 0.25 to 26%.

Suitable allophanate-modified diphenylmethane diisocyanates for preparing these prepolymers for the polyureas (i.e. two-component systems) typically have an NCO group content of from 0.25 to 30% by weight and comprise the reaction product of (a) diphenylmethane diisocyanate, with (b) a polyether monol having an equivalent weight of greater than 1000 to 10,000, in the presence of (c) at least one catalyst. These allophanate-modified diphenylmethane diisocyanates, and components for their preparation, are as described above.

Suitable polyether polyols to be reacted with the allophanate-modified diphenylmethane diisocyanates to prepare the prepolymers are as described herein above with regard to molecular weight, functionality, etc.

In accordance with the present invention, the two-component, amine-cured polyureas comprise the reaction product of (A) a prepolymer of an allophanate-modified diphenylmethane diisocyanate with (B) an isocyanate-reactive component. Suitable components to be used as (B) the isocyanate-reactive component comprise at least one compound selected from the group consisting of (1) one or more amine group containing compound having a functionality of at least about 1.8 and a molecular weight of greater than 750 to about 7000, and (2) at least one diamine or polyamine having a molecular weight of less than or equal to 750. In addition, (B) the isocyanate-reactive component optionally comprises (3) one or more hydroxyl group containing compounds.

Suitable amine group containing compounds having a functionality of at least about 1.8 and a molecular weight of greater than 750 to about 7000 to be used as component (B)(1) herein include compounds such as, for example, amine-terminated polyether polyols, amine terminated silicones, amine-terminated epoxies including those based on 1,2-butylene oxide, amine-terminated polyesters, etc. amine-terminated polyesters, etc. Suitable amine-terminated polyether polyols include those described in, for example, U.S. Patent 6,765,080, the disclosure of which is hereby incorporated by reference. Examples of suitable amine-terminated silicones to be used herein include, for example, those described in, for example, U.S. Published Patent Application 2004/210010, the disclosure of which is hereby incorporated by reference, amine terminated epoxies as described in U.S. Patent 6,723,821, the disclosure of which is hereby incorporated by reference, and amine-terminated 1,2-polyoxybutylene diol as described in U.S. Patent 5,317,076, the disclosure of which is hereby incorporated by reference.

These amine group containing compounds typically have a functionality of from 1.8 to 6. In general, these amine group containing compounds will have a functionality of at least 1.8, and preferably of at least 2. The functionality of suitable amine group containing compounds is typically 6 or less, preferably 4 or less and more preferably 3 or less. Suitable amine group containing compounds may have functionalities ranging between any combination of these functionalities (inclusive), e.g. from 1.8 to 6, preferably from 1.8 to 4, more preferably from 2 to 3, and most preferably 2.

In addition, these amine group containing compounds typically have molecular weights ranging of greater than 750 to 7000. In general, these amine group containing compounds will have molecular weights of greater than 750, preferably at least 1000 and more preferably at least 1500. These amine group containing compounds also typically have molecular weights of less than or equal to 7000, preferably less than or equal to 5000, more preferably less than or equal to 4000 and most preferably less than or equal to 2500. Suitable amine group containing compounds may have molecular weights ranging between any combination of these molecular weights (inclusive, unless otherwise noted), e.g., of greater than 750 to less than or equal to 7000, preferably at least 1000 to less than or equal to 5000, more preferably at least 1000 to less than or equal to 4000, and most preferably 1500 to less than or equal to 2500.

Suitable amine-terminated polyether polyols to be used herein include, for example, those known in the field of polyurethane chemistry. Such amine-terminated polyether polyols include those described in, for example, U.S. Patents 6,635,737, 6,765,080, the disclosures of which are hereby incorporated by reference.

A particularly preferred compound to be used as component (B)(1) herein is an amine-terminated polyether polyol having a functionality of 2 and a molecular weight of about 2000. This polyether polyol is commercially available under the name Jeffamine D-2000 and is available from Huntsman.

Suitable amine group containing compounds having a functionality of at least about 1.8 or more, and a molecular weight of less than or equal to 750 to be used as component (B)(2) herein include compounds such as, for example, lower molecular weight amine-terminated polyether polyols, aromatic and/or (cyclo)aliphatic diamines and polyamines, including the N-alkyl-substituted and N,N'-dialkyl-substituted aromatic diamines, polyaspartic esters, etc. The diamines and polyamines may be primary and/or secondary amine compounds. In general, these amine group containing compounds are known and described, including how to prepare them, in, for example, U.S. Patents 5,126,170, 5,236,741 and 6,765,080, the disclosures of which are herein incorporated by reference.

Suitable diamines and/or polyamines to be used as (B)(2) for the polyureas herein typically have a functionality of from 1.8 to 4. In general, these amine group containing compounds will have a functionality of at least 1.8 and preferably at least 2. The functionality of suitable amine group containing compounds is typically 4 or less, and preferably 3 or less. Suitable diamines and/or polyamines may have functionalities ranging between any combination of these functionalities (inclusive), e.g. from 1.8 to 4, preferably from 2 to 3, and most preferably 2.

In addition, the diamines and/or polyamines to be used as (B)(2) in the polyureas typically have molecular weights less than or equal to 750. In general, these amine compounds will have molecular weights of at least 60, preferably at least 100 and more preferably at least 300. These amine compounds for (B)(2) also typically have molecular weights of less than or equal to 750, preferably less than or equal to 600, and more preferably less than or equal to 400. Suitable amine group containing compounds may have molecular weights ranging between any combination of these molecular weights (inclusive), e.g., from greater than or equal to 60 to less than or equal to 750, preferably greater than or equal to 100 to less than or equal to 600, and more preferably greater than or equal to 300 to less than or equal to 400.

In an optional embodiment, the polyureas additionally comprise (B)(3) one or more hydroxyl group containing compounds. Suitable hydroxyl group containing compounds typically have a functionality of from 1.8 to 6 and a molecular weight of from about 60 to about 10,000. Some examples of suitable hydroxyl group containing compounds include, but are not limited to, polyether polyols, polyester polyols, polythioethers, polyesters, polycaprolactones, polycarbonates, polyacetals, glycols and other relatively low molecular hydroxyl group containing compounds including, for example, ethylene glycol, propylene glycol, butane diol, pentane diol, diethylene glycol, dipropylene glycol, glycerol, pentaerythritol, sorbitol, etc. tripropylene glycol and mixtures thereof.

Suitable hydroxyl group containing compounds to be used as (B)(3) for the polyureas herein typically have a functionality of from 1.8 to 6. In general, these hydroxyl group containing compounds will have a functionality of at least 1.8, and preferably at least 2. The functionality of suitable hydroxyl group containing compounds is typically 6 or less, and preferably 4 or less. Suitable hydroxyl group containing compounds may have functionalities ranging between any combination of these functionalities (inclusive), e.g. from 1.8 to 6, preferably from 2 to 4, and most preferably 2.

In addition, the hydroxyl group containing compounds to be used as (B)(3) in the polyureas typically have molecular weights ranging from 60 to 10,000. In general, these hydroxyl group containing compounds will have molecular weights of at least 60, preferably at least 400, more preferably at least 1000 and most preferably at least 1500. These hydroxyl group containing compounds for (B)(3) also typically have molecular weights of less than or equal to 10,000, preferably less than or equal to 5000, more preferably less than or equal to 4000 and most preferably less than or equal to 2500. Suitable hydroxyl group containing compounds may have molecular weights ranging between any combination of these molecular weights (inclusive), e.g., from greater than or equal to 60 to less than or equal to 10,000, preferably greater than or equal to 400 to less than or equal to 5000, more preferably greater than or equal to 1000 to less than or equal to 4000, and most preferably greater than or equal to 1500 to less than or equal to 2500.

The two-component materials of the present invention may optionally contain one or more catalysts to increase the cure rate. Typically, any of the conventional and well-known catalysts for promoting the urethane reaction are suitable. These include, for example, organometallic catalysts such as those based on tin, mercury, bismuth, zinc, lead, iron, zirconium, titanium, etc. as well as amine catalysts, alkanolamines, etc.

In a particularly preferred embodiment, the sealants are prepared from these polyureas as described above. These sealants are typically two components formulations. One of the components of these polyurea sealants comprises prepolymers of allophanate-modified diphenylmethane diisocyanates. In these sealants, it is preferred that:
(A) said prepolymers of the allophanate modified MDI has an NCO group content of 5 to 26%, preferably 10 to 23% and more preferably 12 to 16%, and comprises the reaction product of;
   (1) an allophanate-modified MDI having an NCO group content of 10 to 30%, preferably 16 to 23% and more preferably 17 to 19%, which comprises the reaction product of
      (a) diphenylmethane diisocyanate having the above described isomer distribution,
         with
      (b) a polyether monol having a molecular weight of greater than 1000 to 10,000, preferably from 1100 to 5000 and more preferably from 1200 to 2000;
      with
   (2) at least one polyether polyol having a functionality of from 1.5 to 6, preferably 1.5 to 3, and more preferably 1.8 to 2.2, and a molecular weight of from 500 to 10,000, preferably 500 to 5000, more preferably 1000 to 3000 and most preferably 1500 to 2500.

For each of the components in these sealants, any combination of the above noted ranges for each of NCO group contents, molecular weights, functionalities, etc., is suitable in accordance with the present invention.

As is known in the art, two-component, amine-cured polyurea sealants are prepared by first preparing a NCO-terminated prepolymer from one or more isocyanates and one or more polyols. This component is used as one of the two components. The second component for the sealant is typically a blend of amine terminated polyether resins, amine terminated chain extenders, optionally polyols, described hereinabove as components (B)(1) through (B)(3), as well as various additives such as, for example, plasticizers, fillers, pigments, light stabilizers, antioxidants, adhesion promoters, and optionally catalysts. The two components are typically mixed thru a static mixtube at a set ratio. The mixed components typically gel in 1 to 60 minutes and harden into a finished sealant. More details concerning sealants and their preparation are described in, for example U.S. Patent 6,635,737, the disclosure of which is herein incorporated by reference.

Suitable one-component, moisture cure, polyureaurethanes of the present invention comprise the reaction product of (A) the above described prepolymer of an allophanate-modified diphenylmethane diisocyanate, and (B) water, optionally, in the presence of (C) one or more catalysts.

In the polyureaurethanes of the present invention, suitable prepolymers of allophanate modified diphenylmethane diisocyanate typically have NCO group contents ranging from 0.25 to 26% and are as described above with respect to the general description of the prepolymers.

Suitable allophanate-modified diphenylmethane diisocyanates for preparing these prepolymers for the one-component polyureaurethanes typically have an NCO group content of from 0.25 to 30% by weight and are as described herein above with regard to the allophanate-modified diphenylmethane diisocyanates. As previously discussed, these comprise the reaction product of (a) diphenylmethane diisocyanates, and preferably in which the isomer distribution is as previously set forth, with (b) a polyether monol having an equivalent weight of greater than 1000 to 10,000, and as described above, in the presence of (c) at least one catalyst.

Suitable polyether polyols for preparing the prepolymers typically have a functionality of from 1.5 to 4 and a molecular weight of from 500 to 10,000. The preferred molecular weight ranges for these polyether polyols are as previously set forth above for preparing the prepolymers of the allophanate-modified diisocyanates.

These polyether polyols will typically have a functionality of from 1.5 to 4. In general, these polyether polyols will have a functionality of at least 1.5, and preferably of at least 1.8. The functionality of suitable polyether polyols is typically 4 or less, preferably 3.5 or less and more preferably 3.2 or less. Suitable polyether polyols may have functionalities ranging between any combination of these functionalities (inclusive), e.g. from 1.5 to 4, preferably from 1.5 to 3.5 and more preferably from 1.8 to 3.2.

As is known in polyurethane chemistry, one-component, moisture-cured polyureaurethanes are prepared by manufacturing a low NCO terminated prepolymer. The prepolymer can be prepared with the fillers and additives present or the prepolymer can be post mixed with the fillers and additives. Typical fillers and additives include carbonates, pigments, plasticizers, adhesion promoters, antioxidants, UV stabilizers, drying agents, crosslinking agents, catalyst, and solvents. In general, additional details are disclosed in, for example, U.S. Published Patent Application 2006/0020101 A1, the disclosure of which is hereby incorporated by reference.

In the one-component, moisture-cure polyureaurethanes of the present invention, the water which reacts with the prepolymer may be moisture from ambient air. Thus, as used in this content, the definition of water includes moisture from ambient air. The one-component materials of this invention are preferably cured in this manner, i.e. with the moisture from ambient air.

In addition, one or more catalysts to facilitate the reaction between the NCO groups of the prepolymer with water (or moisture) can optionally be present. It is preferred that such catalysts are incorporated into the prepolymer. Suitable catalysts here also include the conventional and well-known catalysts for polyurethane and/or polyurea chemistry. Some examples of such catalysts include, in particular, but are not limited to, amine catalysts such as Jeffcat DMDEE.

In the preferred embodiment, caulking agents are prepared from these one-component, moisture-cured polyureaurethanes. In this embodiment, the (A) prepolymer of the allophanate-modified MDI has an NCO group content of from 0.25 to 23% , preferably from 0.5 to 5% and more preferably from 1 to 4%; and comprises the reaction product of (1) an allophanate modified MDI having an NCO group content of from 0.25% to 30%, preferably from 1% to 23%, more preferably from 2 to 12% and most preferably from 4 to 10%, and which is the reaction product of (a) diphenylmethane diisocyanate having the isomer distribution as described above, (b) a polyether monol having a molecular weight of 1000 to 10,000, preferably 2000 to 7000 and more preferably 3500 to 4500 , in the presence of (c) a suitable catalyst; with (2) at least one polyether polyol having a functionality of from 1.5 to 4, preferably 1.5 to 3.5 and more preferably 1.8 to 3.2, and a molecular weight of from 500 to 7000, preferably 1250 to 5000, and more preferably from 1500 to 4500, with 4000 being most particularly preferred.

For each of the components in these caulking agents, any combination of the above noted ranges for each of NCO group contents, molecular weights, functionalities, etc., is suitable in accordance with the present invention.

Caulking agents are prepared as is known in the art. Various processes are known and described in, for example, U.S. Published. Patent Application 2006/0020101 A1.

The following examples further illustrate details for the preparation and use of the compositions of this invention. The invention, which is set forth in the foregoing disclosure, is not to be limited either in spirit or scope by these examples. Those skilled in the art will readily understand that known variations of the conditions and processes of the following preparative procedures can be used to prepare these compositions. Unless otherwise noted, all temperatures are degrees Celsius and all parts and percentages are parts by weight and percentages by weight, respectively.

### EXAMPLES

The following materials and abbreviations are used in the examples:

| | |
|---|---|
| Isocyanate A: | an isomeric mixture of diphenylmethane diisocyanate having an NCO group content of about 33-34% and containing about 57% by weight of the 2,4'-isomer, about 42 % by weight of the 4,4'-isomer and less than 1% by weight of the 2,2'-isomer. |
| Isocyanate B: | an isomeric mixture of diphenylmethane diisocyanate having an NCO group content of about 33-34% and containing about 30% by weight of the 2,4'-isomer, about 70% by weight of the 4,4'-isomer and less than 1% by weight of the 2,2'-isomer. |
| Isocyanate C: | diphenylmethane diisocyanate having an NCO group content of about 33-34% and containing about 98% by weight of the 4,4'-isomer and less than 2% by weight of the 2,2'- and 2,4'-isomers. |
| Alcohol A: | Isobutyl Alcohol (IBA) |
| Monol A: | a monofunctional polyether alcohol having an OH number of about 45, an equivalent weight of about 1250, and comprising the propoxylation product of a mixture of C₁₂ to C₁₅ alcohols. This mixture of C₁₂ to C₁₅ alcohols is commercially available as NEODOL® 25 from Shell Chemical Company. |
| Monol B: | a monofunctional polyether alcohol having an OH number of about 35, an equivalent weight of about 1600, and comprising the propoxylation product of nonylphenol. |
| Monol C: | a monofunctional polyetheralcohol having an OH number of about 36, an equivalent weight of about 1550, and comprising the propoxylation product of a mixture of C₁₂ to C₁₅ alcohols. This mixture of C₁₂ to C₁₅ alcohols is commercially available as NEODOL® 25 from Shell Chemical Company. |
| Monol D: | a monofunctional polyetheralcohol having an OH number of about 165, an equivalent weight of about 340, and comprising the reaction product of propylene oxide with butanol. |
| Monol E: | a monofunctional polyetheralcohol having an OH number of about 15, a theoretical functionality of about 1.2, an equivalent weight of about 4000, and comprising the propoxylation product of Monol C and a small quantity of propylene glycol. A process for preparing this monol is described herein. |
| ZnAcAc: | zinc acetylacetonate, an allophanate catalyst |
| Bz CI: | benzoyl chloride, a catalyst stopper |
| Polyol A: | a polyether polyol having a functionality of 2 and a molecular weight of about 2000, comprising the reaction product of propylene glycol with propylene oxide. |
| Polyol B: | a polyether polyol having a functionality of about 2, an OH number of about 28 and a molecular weight of about 4000, |
| | comprising the reaction product of propylene glycol with propylene oxide. |
| Amine A: | an amine-terminated polyether polyol having a functionality of 2 and a molecular weight of about 2000, commercially available as Jeffamine D-2000 from Hunstman Inc. |
| Amine B: | 4,4'-bis(sec-butylamino)diphenylmethane, an aromatic diamine having a molecular weight of about 310; commercially available as Unilink 4200 from Dorf Ketal Chemicals LLC |
| TiO2: | titanium dioxide, commercially available as Tioxide TR93 from Hunstman, Inc. |
| HALS 1: | bis(1,2,2,6,6-pentamethyl-4-piperidinyl)sebacate, a hindered amine light stabilizer commercially available as Tinuvin 292 from Ciba Geigy |
| HALS 2: | α-[3-[3-(2H-benzotriazol-2-yl)-5-(1,1-dimethyl)-4-hydroxyphenyl]-1-oxopropyl]-ω-hydroxypoly(oxy-1,2-ethanediyl), a hindered amine light stabilizer commercially available as Tinuvin 1130 from Ciba Geigy |
| Irganox 1135: | isooctyl 3-(3,5-di-tert-butyl-4-hydroxyphenyl)propionate, a liquid hindered phenolic antioxidant commercially available as Irganox 1135 from Ciba Geigy |
| Silane A-187: | trimethoxy[3-oxiranylmethoxy)propyl]-silane, an epoxy functional silane commercially available from GE Advanced Materials |

The following procedure was used to prepare Monol E:

5775 g of Monol C were added to a 30 liter agitated reactor. Agitator power input was 8 hp/Mgal. 0.6g of IMPACT-3 catalyst was added which, for a final batch size of 20000g, yields a catalyst concentration of 30 ppm in the product. The starter mixture was de-gassed to remove oxygen, and then heated to the reaction temperature of 130°C. The starter was then vacuum stripped with a nitrogen sparge (100 mmHg vacuum and 40 g/hr nitrogen for 30 minutes). Following the strip, a small amount of PO, corresponding to 231 g or 4% of the starter, was fed to the reactor to activate the catalyst. After the headspace pressure had dropped to half its peak pressure, the PO feed was restarted, and ramped up to a steady state oxide feed rate of 39.9 g/min over 20 minutes. Overall oxide feed time was 6 hrs. During the course of the oxide feed, 39 g of propylene glycol was fed to the reactor. The oxide feed was terminated when 13950g had been fed, not including activation amount. (Including the activation amount, total oxide fed was 14181 g). Following completion of the oxide feed, the reactor was held at reaction temperature for a further 30 minutes to allow the reaction to complete. The reactor was then cooled, and 160 ppm of Vitamin E was added as an inhibitor. Analytical results on the final product (i.e. Monol E) were as follows:

| | |
|---|---|
| OH# | 14.9 mg KOH/g |
| Functionality: | 1.2 |
| Viscosity | 1169 cSt at 25°C |

Each of the prepolymers in Table 1, i.e. Examples 1-9, was prepared in accordance with the following procedure unless otherwise noted.

### EXAMPLES 1- 9: - Prepolymer Viscosity Reduction with Allophanates

To a clean 2-liter, 3-necked round bottom flask was added the required amount of isocyanate. The round bottom flask was equipped with a stirrer, gas bubbler, and thermometer. The isocyanate was heated to 45-50°C with a heating mantle. The required amount of monol was added to the flask at a rate keeping the temperature below 55°C. A water bath was used to cool the reaction if required. The reaction temperature was maintained at 55°C until the theoretical isocyanate content was achieved. Zinc acetylacetonate (Zinc AcAc) was then added to the reactor. The temperature was raised to 70°C and held at that temperature until the theoretical isocyanate value for the allophonate formation was achieved, i.e. referred to as allophanate NCO % herein. A small sample was removed for chemical analysis at this point in the experiment. Benzoyl chloride was added to stop the reaction at the appropriate allophanate NCO%. The amount of isocyanate remaining in the flask was used to calculate the amount of polyol required to achieve the final target NCO content for the prepolymer of the allophanate-modified isocyanate. The final quantity of polyol was added to the round bottom flask. The reaction temperature was maintained at 60°C until the final theoretical NCO content for the prepolymer was achieved. Details concerning the formulations, % NCO and viscosities of the allophanate-modified isocyanates and prepolymers of these are set forth in Table 1.

**Table 1: Effects of Isomer Content and Monol Molecular Weight on Viscosity of Prepolymers of Allophanate-Modified Isocyanates**

| Example | 1 | 2 | 3 | 4 | 5 | 6 | 7 | 8 | 9 |
|---|---|---|---|---|---|---|---|---|---|
| Iso A | | | 58.32 | 52.89 | | | 49.81 | 49.31 | 49.41 |
| Iso B | | 58.58 | | | | 49.50 | | | |
| Iso C | 58.60 | | | | 49.58 | | | | |
| Alcohol A | 3.2 | 3.19 | 3.18 | | | | | | |
| Monol A | | | | | 11.32 | 11.30 | 11.56 | | |
| Monol B | | | | | | | | 11.94 | |
| Monol C | | | | | | | | | 12.07 |
| Monol D | | | | 7.70 | | | | | |
| ZnAcAc | 100 ppm | 100 ppm | 100 ppm | 100 ppm | 100 ppm | 100 ppm | 100 ppm | 100 ppm | 100 ppm |
| Bz Cl | 200 ppm | 200 ppm | 200 ppm | 200 ppm | 200 ppm | 200 ppm | 200 ppm | 200 ppm | 200 ppm |
| % NCO (Alloph.) | 25.7% | 25.7% | 25.8% | 25.96% | 25.68% | 25.73% | 25.78% | 25.84% | 25.73% |
| Viscosity * - Allophonate | 81 | 114 | 146 | 30.5 | Solid | 10.8 | 30.5 | 27.5 | 19.7 |
| Polyol A | 38.20 | 28.23 | 38.50 | 39.41 | 39.10 | 39.20 | 38.63 | 38.75 | 38.52 |
| % NCO (Prepolymer) | 14.2% | 14.2% | 14.2% | 14.0% | 13.9% | 13.7% | 14.1% | 14.1% | 14.2% |
| Viscosity* - Prepolymer | 2110 | 2100 | 2405 | 1185 | 793 | 838 | 908 | 964 | 905 |

| | | | | | | | | | |
|---|---|---|---|---|---|---|---|---|---|
| * viscosity in cps | | | | | | | | | |

EXAMPLES 10 thru 15: These examples describe two component polyureas which are suitable as sealants. Each of the sealant formulations in Table 2, i.e. Examples 10-15, was prepared in accordance with the following procedure unless otherwise noted.

### EXAMPLES 10-15:

The Part B components as set forth in Table 2 were weighed into a 400 g maximum plastic Flak Tek cup. The cup was spun for 1 minute at 2,000 rpm. The required amount of Part A (i.e. the prepolymer of the allophanate-modifed MDI from Examples 1-3, or 7-9) was then added to the Flak Tek cup. A Gardner gel timer was started at the same time the Flak Tek mixer was started. The Flak Tek cup was spun for 30 seconds at 2,000 rpm. The mixed resin was poured into an aluminum cup. The cup was placed into the Gardner gel timer. The gel time was measured when the Gardner gel timer stopped spinning. A sample for physical property determination was prepared in the same fashion. However, the mixed resin was poured into an 8 x 10 x ¼ inch window mold. The samples were allowed to cure at room temperature. The physical properties were determined and set forth in Table 2.

**Table 2. Examples 10-15 - Effects of Isomer Content and Monol Molecular Weight on Properties of Polyurea Sealants**

| Example | 10 | 11 | 12 | 13 | 14 | 15 |
|---|---|---|---|---|---|---|
| B-Side | | | | | | |
| Amine A | 22.27 | 22.27 | 22.27 | 22.27 | 22.27 | 22.26 |
| Amine B | 20.51 | 20.51 | 20.51 | 20.51 | 20.51 | 20.50 |
| TiO2 | 4.52 | 4.52 | 4.52 | 4.52 | 4.52 | 4.52 |
| HALS 1 | 0.23 | 0.23 | 0.23 | 0.23 | 0.23 | 0.23 |
| HALS 2 | 0.23 | 0.23 | 0.23 | 0.23 | 0.23 | 0.23 |
| Irganox 1135 | 0.45 | 0.45 | 0.45 | 0.45 | 0.45 | 0.45 |
| Silane A-187 | 0.23 | 0.23 | 0.23 | 0.23 | 0.23 | 0.23 |

| Isocyanate | | | | | | |
|---|---|---|---|---|---|---|
| Prep. Ex. 1 | 51.57 | | | | | |
| Prep. Ex. 2 | | 51.57 | | | | |
| Prep. Ex. 3 | | | 51.57 | | | |
| Prep. Ex. 7 | | | | 51.57 | | |
| Prep. Ex. 8 | | | | | 51.57 | |
| Prep. Ex. 9 | | | | | | 51.58 |
| | | | | | | |

| Properties | | | | | | |
|---|---|---|---|---|---|---|
| Gel (mins) | 2.42 | 3.18 | 4.38 | 5.45 | 4.51 | 5.43 |
| Tensile (psi) | 1692 | 1752 | 1872 | 1184 | 1009 | 864 |
| 100% Modulus (psi) | 1915 | 1584 | 1244 | 600 | 511 | 441 |
| 200% Modulus (psi) | | 1719 | 1290 | 676 | 572 | 780 |
| 300% Modulus (psi) | | 614 | 1453 | 783 | 664 | 535 |
| Elongation (%) | 210 | 131 | 248 | 528 | 518 | 616 |
| Tear (pli) | 403 | 530 | 575 | 313 | 294 | 265 |
| Shore A | 100 | 67 | 100 | 94 | 93 | 83 |
| Shore A (5 sec) | 100 | 67 | 100 | 85 | 85 | 81 |

### EXAMPLE 16: Example 16 is a prepolymer of Isocyanate A. This prepolymer was prepared by the following procedure:

To a clean 2-liter, 3-necked round bottom flask was added the required amount of isocyanate. The round bottom flask was equipped with a stirrer, gas bubbler, and thermometer. The isocyanate was heated to 60 C with a heating mantle. The polyol was added to the reactor with stirring while the temperature was maintained at 60 C. The reaction temperature was maintained until the final theoretical NCO content was achieved. The formulation is set forth in Table 3.

EXAMPLES 17 thru 20: These examples represent an allophanate-modified isocyanate (Example 20) and prepolymers of allophanate-modified isocyanates (Examples 17-19). The prepolymers were prepared by the same procedures as set forth above for Examples 1-9. The allophanate-modifed isocyanate in Example 20 was prepared by a similar procedure as described for Examples 1-9 but the procedure ended with the addition of the benzoyl chloride. The formulation details are set forth in Table 3.

EXAMPLE 21: This example represents a prepolymer of a conventional allophanate-modified isocyanate in which the allophanate-modified isocyanate is the reaction product of diphenylmethane diisocyanate and an aliphatic alcohol (i.e. isobutyl alcohol). This example was prepared by the same procedure as set forth above for Examples 1-9. The specific formulation is set forth in Table 3.

**Table 3: Examples 16-21 - Effect of Allophonate Content on Viscosity**

| Example | 16 | 17 | 18 | 19 | 20 | 21 |
|---|---|---|---|---|---|---|
| Iso A | 48.80 | 49.81 | 50.27 | 51.10 | 51.41 | 63.16 |
| Alcohol A | | | | | | 4.91 |
| Monol A | | 11.56 | 17.92 | 31.89 | 48.59 | |
| Zn AcAc | | 100 PPM | 100 PPM | 100 PPM | 100 PPM | 100 PPM |
| Bz Cl | | 200 PPM | 200 PPM | 200 PPM | 200 PPM | 200 PPM |
| % NCO (Alloph.) | ---- | 25.78 | 22.79 | 17.85 | 13.88 | 22.8 |
| Viscosity * Alloph. | ---- | 30.5 | 66.8 | 219 | 455 | 777 |
| Polyol A | 51.20 | 38.63 | 31.82 | 17.01 | ---- | 31.93 |
| % NCO (Prepol.) | 14.3 | 14.12 | 14.15 | 14.08 | ---- | 14.10 |
| Viscosity* Prepol. | 1004 | 908 | 770 | 516 | ---- | 4743 |
| Wt. % Allophon. | 0 | 16 | 25 | 45 | 68 | 38 |

| | | | | | | |
|---|---|---|---|---|---|---|
| * viscosity in cps | | | | | | |

EXAMPLES 22-27: These Examples demonstrate the preparation of two-component polyurea sealants. Example 22 is a comparative example of a two-component polyurea sealant which was prepared from the prepolymer of the allophanate-modified isocyanate from Example 16 in Table 3 above. Examples 23-26 are representative of the two-component polyurea sealants of the present invention. Examples 23-27 use the compositions prepared in Examples 17-20 from Table 3. The formulations for these two-component sealants are set forth in Table 4. The B-side components in Table 4 were weighed into a 400 g maximum plastic Flak Tek cup. The cup was spun for 1 minute at 2,000 rpm. The required amount of A-side (i.e. allophanate-modified isocyanates, prepolymers of allophanate-modified isocyanates, and isocyanate prepolymers) was then added to the Flak Tek cup. A Gardner gel timer was started at the same time the Flak Tek mixer was started. The Flak Tek cup was spun for 30 seconds at 2,000 rpm. The mixed resin was poured into an aluminum cup. The cup was placed into the Gardner gel timer. The gel time was measured when the Gardner gel timer stopped spinning. A sample for physical property determination was prepared in the same fashion. However, the mixed resin was poured into an 8 x 10 x ¼ inch window mold. The samples were allowed to cure at room temperature. The physical properties were determined and are set forth in Table 4.

**Table 4. Effects of Allophonate Content on Physical Properties of Polyurea Sealants**

| Example | 22 | 23 | 24 | 25 | 26 | 27 |
|---|---|---|---|---|---|---|
| Part B | | | | | | |
| Amine A | 22.27 | 22.27 | 22.27 | 22.27 | 22.27 | 22.27 |
| Amine B | 20.51 | 20.51 | 20.51 | 20.51 | 20.51 | 20.51 |
| TiO2 | 4.52 | 4.52 | 4.52 | 4.52 | 4.52 | 4.52 |
| HALS 1 | 0.23 | 0.23 | 0.23 | 0.23 | 0.23 | 0.23 |
| HALS 2 | 0.23 | 0.23 | 0.23 | 0.23 | 0.23 | 0.23 |
| Irganox 1135 | 0.45 | 0.45 | 0.45 | 0.45 | 0.45 | 0.45 |
| Silane A-187 | 0.23 | 0.23 | 0.23 | 0.23 | 0.23 | 0.23 |

| Isocyanate | | | | | | |
|---|---|---|---|---|---|---|
| Prep. Ex. 16 | 51.57 | | | | | |
| Prep. Ex. 18 | | | 51.57 | | 25.79 | |
| Prep. Ex. 17 | | 51.57 | | | | |
| Prep. Ex. 19 | | | | 51.57 | | |
| Alloph. Iso. Ex. 20 | | | | | 25.79 | 51.57 |

| Properties | | | | | | |
|---|---|---|---|---|---|---|
| Gel (mins) | 5.35 | 5.45 | 5.80 | 6.65 | ND | 4.92 |
| Tensile (psi) | 944 | 1184 | 1410 | 1397 | 1385 | 687 |
| 100% Modulus (psi) | 319 | 600 | 578 | 697 | 661 | 462 |
| 200% Modulus (psi) | 397 | 676 | 645 | 748 | 708 | 455 |
| 300% Modulus (psi) | 484 | 783 | 744 | 847 | 798 | 474 |
| Elong. (%) | 690 | 528 | 608 | 552 | 583 | 627 |
| Tear (pli) | 248 | 313 | 303 | 368 | 373 | 277 |
| Shore A | 76 | 94 | 95 | 96 | 96 | 94 |
| Shore A (5 sec) | 68 | 85 | 85 | 96 | 95 | 89 |

EXAMPLE 29: This example illustrates one-component, low NCO content, moisture-curing prepolymers. Example 29 illustrates the physical properties of one component, moisture curing base resin. This resin could be blended with fillers and additives to make a caulking agent.

### Examples 28 and 29 were prepared by the following procedure:

To a clean 2-liter, 3-necked round bottom flask was added the required amount of isocyanate. The round bottom flask was equipped with a stirrer, gas bubbler, and thermometer. The isocyanate was heated to 45-50°C with a heating mantle. The required amount of a 4000 molecular weight monol was added to the flask at a rate keeping the temperature below 55°C. A water bath was used to cool the reaction if required. The reaction temperature was maintained at 55°C until the theoretical isocyanate content was achieved. Zinc AcAc was then added to the reactor. The temperature was raised to 70°C and held at that temperature until the theoretical isocyanate value for allophanate formation was achieved. A small sample was removed for chemical analysis at this point in the experiment. Benzoyl chloride was added to stop the reaction. The amount of isocyanate remaining in the flask was used to calculate the amount of polyol required to achieve the final target NCO content. The polyol was added to the flask while the temperature was held at 60°C. The reaction temperature was maintained until the final theoretical NCO content was achieved. The formulations are set forth in Table 5.

Control EXAMPLE 30: Example 30 is a prepolymer of diphenylmethane diisocyanate. To a clean 2-liter, 3-necked round bottom flask was added the required amount of isocyanate. The round bottom flask was equipped with a stirrer, gas bubbler, and thermometer. The isocyanate was heated to 60°C with a heating mantle. The polyol was added to the reactor with stirring. The reaction temperature was maintained until the final theoretical NCO content was achieved. The formulation is set forth in Table 5.

**Table 5. Effects of Allophonate Content on Viscosity and Physical Properties of Prepolymers**

| Example | 28 | 29 | 30 |
|---|---|---|---|
| Iso A | 15.16 | 14.33 | 15.37 |
| Monol E | 84.84 | 41.61 | ---- |
| % NCO (Alloph.) | 3.52 | 7.15 | ---- |
| Viscosity - Alloph. (cps) | 806 | 2490 | ---- |
| Zn AcAc | 100 ppm | 100 ppm | ---- |
| Bz Cl | 200 ppm | 200 ppm | ---- |
| Polyol B | ---- | 44.05 | 84.63 |
| % NCO (Prepol.) | ---- | 3.25 | 3.33 |
| Viscosity - Prepol. (cps) | ---- | 5341 | 10862 |
| Tensile (psi) | 264 | 570 | 1490 |
| 100% Modulus (psi) | 70 | 204 | 360 |
| 200% Modulus (psi) | 150 | 303 | 489 |
| 300% Modulus (psi) | 245 | 373 | 586 |
| Elongation (%) | 324 | 659 | 966 |
| Tear (pli) | 37 | 103 | 215 |

| | | | |
|---|---|---|---|
| (1) mechanical properties in the materials set forth in Table 5 were measured on moisture cured films at ambient conditions | | | |

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

## Claims

1. An allophanate-modified diphenylmethane diisocyanate having an NCO group content of 0.25 to 30%, and comprising the reaction product of:
(a) diphenylmethane diisocyanate,
with
(b) a polyether monol having an equivalent weight of greater than 1000 up to about 10,000;
in the presence of:
(c) a suitable catalyst.

2. The allophanate-modified diphenylmethane diisocyanate of Claim 1 having an NCO group content of from 1 to 26%, wherein
(a) said diphenylmethane diisocyanate comprises from 1 to 81% by weight of the 2,4'-isomer, from 19 to 99% by weight of the 4,4'-isomer and from 0 to 6% by weight of the 2,2'-isomer, with the sum of the %'s by weight of the isomers totaling 100% by weight of said diphenylmethane diisocyanate;
(b) said polyether monol has an equivalent weight of greater than 1000 to 10,000.

3. A process for the preparation of the allophanate-modified diphenylmethane diisocyanate of Claim 1, comprising reacting
(a) diphenylmethane diisocyanate, with
(b) a polyether monol having an equivalent weight of greater than 1000 up to about 10,000;
in the presence of:
(c) a suitable catalyst.

4. A prepolymer of an allophanate-modified diisocyanate having an NCO group content of 0.25 to 26% and comprising the reaction product of:
(1) an allophanate-modified diphenylmethane diisocyanate having an NCO group content of 0.25 to 30% and which comprises the reaction product of:
(a) diphenylmethane diisocyanate,
with
(b) a polyether monol having an equivalent weight of greater than 1000 up to about 10,000;
in the presence of
(c) a suitable catalyst;
and
(2) at least one polyether polyol having a functionality of from 1.5 to 6 and a molecular weight of from 500 to 10,000.

5. The prepolymer of Claim 4 having an NCO group content of 0.5 to 23%, wherein
(1) said allophanate-modified diphenylmethane diisocyanate has an NCO group content of 1 to 26%, and comprises the reaction product of:
(a) diphenylmethane diisocyanate comprising from 1 to 81% by weight of the 2,4'-isomer, from 19 to 99% by weight of the 4,4'-isomer and from 0 to 6% by weight of the 2,2'-isomer, with the sum of the %'s by weight of the isomers totaling 100% by weight of said diphenylmethane diisocyanate;
and
(b) a polyether monol having an equivalent weight of from 1000 to 10,000;
in the presence of
(c) a suitable catalyst;
and
(2) said polyether polyol has a functionality of from 1.5 to 6 and a molecular weight of from 500 to 10,000.

6. A process for the preparation of the prepolymer of the allophanate-modified diphenylmethane diisocyanate having an NCO group content of 0.25 to 26% of Claim 4, comprising reacting
(1) an allophanate-modified diphenylmethane diisocyanate having an NCO group content of 0.25 to 30% and which comprises the reaction product of:
(a) diphenylmethane diisocyanate, with
(b) a polyether monol having an equivalent weight of greater than 1000 up to about 10,000;
in the presence of
(c) a suitable catalyst;
and
(2) at least one polyether polyol having a functionality of from 1.5 to 6 and a molecular weight of from 500 to 10,000.

7. A two-component polyurea comprising the reaction product of:
(A) the prepolymer of Claim 4;
and
(B) an isocyanate-reactive component comprising at least one compound selected from the group consisting of:
(1) one or more amine group containing compound having a functionality of at least 1.8 and a molecular weight of greater than 750 to about 7000;
and
(2) at least one diamine or polyamine having a functionality of at least 1.8 and a molecular weight of less than or equal to 750.

8. The two-component polyurea of Claim 7, wherein
(A) said prepolymer of the allophanate-modified diphenylmethane diisocyanate has an NCO group content of 0.25 to 26% by weight and comprises the reaction product of:
(1) an allophanate-modified diphenylmethane diisocyanate having an NCO group content of 1 to 26% by weight and which comprises the reaction product of:
(a) diphenylmethane diisocyanate,
with
(b) a polyether monol having an equivalent weight of greater than 1100 up to about 7000;
in the presence of
(c) a suitable catalyst;
and
(2) at least one polyether polyol having a functionality of from 1.5 to 4 and a molecular weight of from 1000 to 7000.

9. A two-component polyurea comprising the reaction product of:
(A) a prepolymer of an allophanate-modified diphenylmethane diisocyanate which has an NCO group content of 5 to 26% by weight and comprises the reaction product of:
(1) an allophanate-modified diphenylmethane diisocyanate having an NCO group content of 10 to 30% by weight and which comprises the reaction product of:
(a) diphenylmethane diisocyanate which comprises from 1 to 81 % by weight of the 2,4'-isomer, from 19 to 99% by weight of the 4,4'-isomer and from 0 to 6% by weight of the 2,2'-isomer, with the sum of the %'s by weight of the isomers totaling 100% by weight of said diphenylmethane diisocyanate;
with
(b) a polyether monol having an equivalent weight of from 1100 up to 5,000;
in the presence of
(c) a suitable catalyst;
and
(2) at least one polyether polyol having a functionality of from 1.5 to 6 and a molecular weight of from 500 to 10,000;
and
(B) said isocyanate-reactive component comprising at least one compound selected from the group consisting of:
(1) one or more amine group containing compound having a functionality of at least 1.8 and a molecular weight of greater than 750 up to about 7000;
and
(2) at least one diamine or polyamine having a functionality of at least 1.8 and a molecular weight of less than or equal to 750.

10. The two-component polyurea of Claim 7, wherein (B) said isocyanate-reactive component additionally comprises (3) one or more hydroxyl group containing compounds

11. A process for preparing a polyurea, comprising reacting:
(A) the prepolymer of Claim 4;
with
(B) an isocyanate-reactive component comprising at least one compound selected from the group consisting of:
(1) one or more amine group containing compound having a functionality of at least 1.8 and a molecular weight of greater than 750 to about 5000;
and
(2) at least one diamine or polyamine having a functionality of at least 1.8 and a molecular weight of less than or equal to 750.

12. A one-component polyureaurethane comprising the reaction product of:
(A) the prepolymer of Claim 4;
with
(B) water;
optionally, in the presence of
(C) one or more catalysts.

13. The one-component polyureaurethane of Claim 12, wherein
(A) said prepolymer of an allophanate-modified diisocyanate has an NCO group content of 0.5 to 23% and comprises the reaction product of:
(1) an allophanate-modified diphenylmethane diisocyanate having an NCO group content of 1 to 26% and which comprises the reaction product of:
(a) diphenylmethane diisocyanate comprising (i) from 20 to 73% by weight of the 2,4'-isomer, (ii) from 27 to 80% by weight of the 4,4'-isomer, and (iii) from 0 to 3% by weight of the 2,2'-isomer, with the sum of the %'s by weight of (i), (ii) and (iii) totaling 100% by weight of diphenylmethane diisocyanate,
with
(b) a polyether monol having an equivalent weight of greater than 1100 up to about 7000;
in the presence of
(c) a suitable catalyst;
and
(2) at least one polyether polyol having a functionality of from 1.5 to 3.5 and a molecular weight of from 1000 to 7000.

14. A one-component polyureaurethane which comprises
(A) a prepolymer of an allophanate-modified diphenylmethane diisocyanate which has an NCO group content of 0.25 to 23% and comprises the reaction product of:
(1) an allophanate-modified diphenylmethane diisocyanate having an NCO group content of 0.25 to 30% and which comprises the reaction product of:
(a) diphenylmethane diisocyanate which comprises from 1 to 81% by weight of the 2,4'-isomer, from 19 to 99% by weight of the 4,4'-isomer and from 0 to 6% by weight of the 2,2'-isomer, with the sum of the %'s by weight of the isomers totaling 100% by weight of said diphenylmethane diisocyanate;
with
(b) a polyether monol having an equivalent weight of 1000 up to about 10,000;
in the presence of
(c) a suitable catalyst;
and
(2) at least one polyether polyol having a functionality of from 1.5 to 4 and a molecular weight of from 500 to 7000;
and
(B) water;
optionally, in the presence of
(C) one or more catalysts.

15. A process of preparing a polyureaurethane comprising reacting:
(A) the prepolymer of Claim 4;
and
(B) water;
optionally in the presence of
(C) one or more catalysts.

16. A sealant comprising the two-component polyurea of Claim 9.

17. A caulking agent comprising the one-component polyureaurethane of Claim 14.
